# EUROPÄISCHE PATENTSCHRIFT

(11) **EP 1 322 321 B1**
(45) Veröffentlichungstag und Bekanntmachung des Hinweises auf die Patenterteilung: **25.06.2008**
(21) Anmeldenummer: 01986301.8
(22) Anmeldetag: 02.10.2001
(51) Int. Cl.: A61K 38/03, A61K 38/10, A61P 11/00, A61P 35/00, A61P 31/00

(54) **Verwendung von S-[2,3-Bispalmitoyloxy-(2RS)-propyl]cysteinyl-GNNDESNISFKEK (MALP-2)**
Use of S-[2,3-Bispalmitoyloxy-(2RS)-propyl]cysteinyl-GNNDESNISFKEK (MALP-2)
Utilisation de S-[2,3-Bispalmitoyloxy-(2RS)-propyl]cysteinyl-GNNDESNISFKEK (MALP-2)

(30) Priorität: 02.10.2000 DE 10048840
(43) Veröffentlichungstag der Anmeldung: 02.07.2003
(73) Patentinhaber: Helmholtz-Zentrum für Infektionsforschung GmbH, 38124 Braunschweig (DE)
(72) Erfinder: MÜHLRADT, Peter, 38114 Braunschweig (DE); LÜHRMANN, Anke, 30449 Hannover (DE); TSCHERNIG, Thomas, 31139 Hildesheim (DE); PABST, Reinhard, 30627 Hannover (DE)
(74) Vertreter: Boeters, Hans Dietrich
(86) Internationale Anmeldenummer: PCT/EP2001/011414
(87) Internationale Veröffentlichungsnummer: WO 2002/028887

(56) Entgegenhaltungen:
- EP-A- 0 000 330
- WO-A-99/59610
- DE-A- 19 652 586
- PATENT ABSTRACTS OF JAPAN vol. 016, no. 338 (C-0965), 22. Juli 1992 (1992-07-22) & JP 04 099796 A (KAZUO ACHINAMI;OTHERS: 01), 31. März 1992 (1992-03-31)
- KAUFMANN A ET AL: "INDUCTION OF CYTOKINES AND CHEMOKINES IN HUMAN MONOCYTES BY MYCOPLASMA FERMENTANS-DERIVED LIPOPROTEIN MALP-2." INFECTION AND IMMUNITY, Bd. 67, Nr. 12, Dezember 1999 (1999-12), Seiten 6303-6308, XP002203724 ISSN: 0019-9567
- DEITERS U ET AL: "MYCOPLASMAL LIPOPEPTIDE MALP-2 INDUCES THE CHEMOATTRACTANT PROTEINS MACROPHAGE INFLAMMATORY PROTEIN 1 ALPHA (MIP-1 ALPHA), MONOCYTE CHEMOATTRACTANT PROTEIN 1, AND MIP-2 AND PROMOTES LEUKOCYTE INFILTRATION IN MICE" INFECTION AND IMMUNITY, AMERICAN SOCIETY FOR MICROBIOLOGY. WASHINGTON, US, Bd. 67, Nr. 7, Juli 1999 (1999-07), Seiten 3390-3398, XP002123775 ISSN: 0019-9567
- GARCIA JOSEFINA ET AL: "A MYCOPLASMA FERMENTANS-DERIVED SYNTHETIC LIPOPEPTIDE INDUCES AP-1 AND NF-KAPPAB ACTIVITY AND CYTOKINE SECRETION IN MACROPHAGES VIA THE ACTIVATION OF MITOGEN-ACTIVATED PROTEIN KINASE PATHWAYS." JOURNAL OF BIOLOGICAL CHEMISTRY, Bd. 273, Nr. 51, 18. Dezember 1998 (1998-12-18), Seiten 34391-34398, XP002203725 ISSN: 0021-9258
- SADEK MOHAMED I ET AL: "CHEMOKINES INDUCED BY INFECTION OF MONONUCLEAR PHAGOCYTES WITH MYCOBACTERIA AND PRESENT IN LUNG ALVEOLI DURING ACTIVE PULMONARY TUBERCULOSIS." AMERICAN JOURNAL OF RESPIRATORY CELL AND MOLECULAR BIOLOGY, Bd. 19, Nr. 3, 1998, Seiten 513-521, XP002203726 ISSN: 1044-1549
- HOLGATE STEPHEN T ET AL: "RELEASE OF RANTES, MIP-1ALPHA, AND MCP-1 INTO ASTHMATIC AIRWAYS FOLLOWING ENDOBRONCHIAL ALLERGEN CHALLENGE." AMERICAN JOURNAL OF RESPIRATORY AND CRITICAL CARE MEDICINE, Bd. 156, Nr. 5, November 1997 (1997-11), Seiten 1377-1383, XP002203727 ISSN: 1073-449X
- MICHALEC LIDIA ET AL: "DEFINING THE ROLE OF CC AND CXC CHEMOKINES THAT MEDIATE AIRWAY INFLAMMATION FROM OZONE-INDUCED OXIDATIVE LUNG INJURY." JOURNAL OF ALLERGY AND CLINICAL IMMUNOLOGY., Bd. 105, Nr. 1 PART 2, Januar 2000 (2000-01), Seiten S124-S125, XP002203728 ISSN: 0091-6749

## Beschreibung

Die Erfindung betrifft die Verwendung von S-[2,3-Bispalmitoyloxy-(2RS)-propyl]cysteinyl-GNNDESNISFKEK (MALP-2) zur Herstellung eines Mittels zur Behandlung von Lungenmetastasen extrapulmonaler Tumoren.

### Einleitung

Es ist schon seit längerer Zeit bekannt, daß ursprünglich aus Mikroorganismen isolierte und synthetisch verfügbare Lipopeptide Makrophagen aktivieren (Baschang, G.. 1989. Tetrahedron 45:6331-6360.). In neuerer Zeit sind Varianten solcher Lipopeptide aus Mykoplasmen bekannt geworden, die aufgrund besonderer Strukturmerkmale besonders aktiv sind (Mühlradt, P. F., M. Kieß, H. Meyer, R. Süßmuth, und G. Jung. 1997. J. Exp. Med. 185:1951-1958.; Mühlradt, P. F., M. Kieß, H. Meyer, R. Süßmuth und G. Jung. 1998. Infect. Immun. 66: 9804-4810). Derartige Lipopeptide sind synthetisch erhältlich (Metzger, J. W., K.-H. Wiesmüller und G. Jung. 1991. Int. J. Peptide Protein. Res. 38: 545-554). Sie sind dazu fähig, in Zellkulturen bei pg/ml-Konzentrationen diverse Zellen, darunter in erster Linie Makrophagen, zur Synthese von proinflammatorischen Zytokinen (Interleukin-1, Interleukin-6, Tumor-Nekrose-Faktor) und Chemokinen (MIP-1, MIP-2, MCP-1, IL-8) anzuregen (Deiters, U. und P. F. Mühlradt. 1999. Infect. Immun. 67: 3390-3398; Kaufmann, A., P. F. Mühlradt, D. Gemsa und H. Sprenger. 1999. Infect. Immun. 67: 6303-6308).

Es ist weiterhin bekannt, daß der physiologische Rezeptor dieser Lipopeptide der "Toll-like"-Rezeptor 2 ist, und daß vorzugsweise Lipopeptide mit dem natürlichen Enantiomer des Lipidteils aktiv sind (Takeuchi, A., A. Kaufmann, K. Grote, T. Kawai, K. Hoshino, M. Morr, P. F. Mühlradt und S. Akira. 2000. J. Immunol. 164: 554-557).

An eine therapeutische Verwendung dieser Substanzen konnte bisher nicht gedacht werden, da die klassischen bakteriellen Lipoproteine im Tierversuch praktisch inaktiv waren (Hauschildt, S., H. U. Beuscher, G. Jung, W. Bessier und A. Ulmer. 1994. FEMS Immunol. Med. Microbiol. 8:77-82). Das aus *Mykoplasma fermentans* isolierte, Makrophagen aktivierende Lipopeptid MALP-2 (ein S-(2,3-Bisacyloxypropyl)-cysteinpeptid) ist jedoch im Tierversuch aktiv in dem Sinn, daß eine intraperitoneale Injektion von wenigen µg eine Infiltration von Leukozyten bewirkte (Deiters, U. und P. F. Mühlradt. 1999. Infect. Immun. 67: 3390-3398).

Es ist bisher nicht möglich gewesen, mit untoxischen Mitteln eine gezielte Infiltration von Leukozyten in die Lunge zu erzielen. Dies kann wünschenswert sein, um z.B. chronische Infekte zu bekämpfen oder inoperable Tumore zu therapieren. Eine entsprechende Therapie bei der Behandlung von Blasentumoren besteht darin, Makrophagen dadurch anzulocken und zu aktivieren, daß man lebende Mykobakterien in die Blase injiziert (Zlotta A. R, J. P. Van Vooren, O. Denis, A. Drowart, M. Daffe, P. Lefevre, L. Schandene, M. De Cock, J. De Bruyn, P. Vandenbussche, F. Jurion, K. Palfliet, J. Simon, C. C. Schulman, J. Content, K. Huygen. 2000. Int. J. Cancer 87: 844-52). Die inhalative Applikation des Lymphokins Interleukin-2 führt unter geringen Nebenwirkungen zur Reduktion von Tumormetastasen in der Lunge (Huland, E., H. Heinzer, H. Huland, R. Yung. 2000. Cancer J. Sci. Am. 6: Suppl.1, 104-112).

Ferner ist aus WO 99/59 610 die Verwendung eines Lipopeptids zur Tier- oder Human-Wundbehandlung bekannt. Nach diesem Stand der Technik kann das Einströmen von Leukozyten in die Lunge bei Infektion mit Hilfe von Mykoplasmen durch die in den Mykoplasmen vorhandenen Lipopeptide hervorgerufen werden.

Schließlich offenbart DE 196 52 586 bereits die Verwendung von S-(2,3-Dihydroxypropyl)-cystein-peptid zur Vorbeugung allgemein gegen Infektionen oder als Immunstimulanz gegen Tumoren und ferner zur Stimulierung der Antikörper-Synthese bzw. zur Aktivierung von Makrophagen.

Die Erfindung beruht auf der überraschenden Erkenntnis, daß S-[2,3-Bispalmitoyloxy-(2RS)-propyl]cysteinyl-GNNDESNISFKEK (MALP-2) zur Herstellung eines Mittels zur Behandlung von Lungenmetastasen extrapulmonaler Tumoren vorgesehen werden kann.

Zur erfindungsgemäßen Verwendung kann MALP-2 in Form einer zur Inhalation geeigneten wäßrigen Lösung, Suspension oder Emulsion vorliegen.

Die erfindungsgemäße Verwendung eignet sich zur Behandlung von Lungenmetastasen extrapulmonaler Tumoren. Ohne sich auf eine bestimmte Theorie festlegen zu wollen, wird angenommen, daß der Wirkmechanismus auf der Rekrutierung und Aktivierung von Abwehrzellen, insbesondere der Auslösung einer Leukozyteninfiltration in die Lunge, beruht.

Nachstehend wird die Erfindung anhand von Beispielen ohne Beschränkung detaillierter beschrieben.

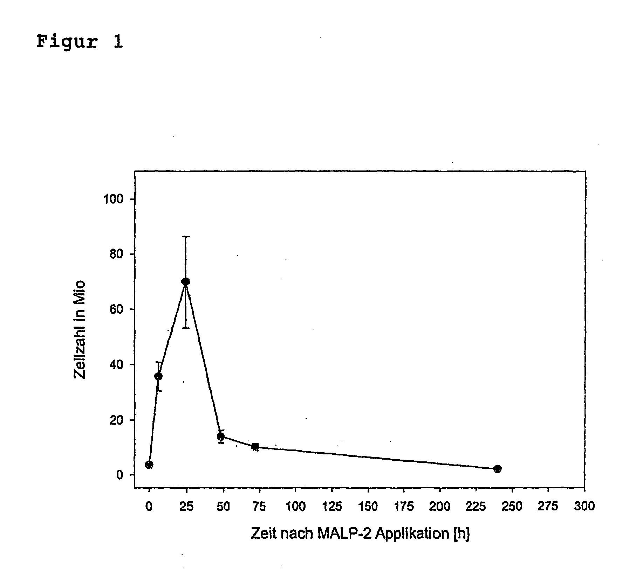

Figur 1 zeigt die Kinetik des Anstiegs der Leukozytenzahl in der bronchoalveolären Lavage nach Applikation von 2,5 µg MALP-2.

### Beispiele

1) Ratten werden 3 µg MALP-2 (ein S-(2,3-Bisacyloxypropyl)-cysteinpeptid) in die Trachea instilliert. Zu unterschiedlichen Zeiten nach der Behandlung werden Zellen aus dem Lungen-Lumen mittels einer bronchoalveolären Lavage (BAL) bestimmt. Die MALP-2-Behandlung führt nach wenigen Stunden zu einem Anstieg der Anzahl von Leukozyten in den Luftwegen und dem Alveolarraum dieser Tiere, der einige Tage anhält (Figur 1). Eine Beeinträchtigung der Tiere durch die MALP-2-Applikation war nicht erkennbar. Nahrungsaufnahme, Körpergewicht, Aktivität und Verhalten waren unauffällig.
2) Ratten läßt man über eine Maske sechsmal im Abstand von je einer Woche Dosen á 30 µg MALP-2 inhalieren. Dies führt zur Zunahme des lymphatischen Gewebes in der Bronchialschleimhaut. Auch bei diesem Versuch waren die Tiere klinisch unauffällig.

## Patentansprüche

1. Verwendung von S-[2,3-Bispalmitoyloxy-(2RS)-propyl]cysteinyl-GNNDESNISFKEK (MALP-2) zur Herstellung eines Mittels zur Behandlung von Lungenmetastasen extrapulmonaler Tumoren.

2. Verwendung nach Anspruch 1, wobei MALP-2 in Form einer zur Inhalation geeigneten wäßrigen Lösung, Suspension oder Emulsion vorliegt.

## Claims

1. Use of S-[2,3-bispalmitoyloxy-(2RS)-propyl]cysteinyl-GNNDESNISFKEK (MALP-2) for preparation of a composition for treating lung metastases of extrapulmonary tumours.

2. Use according to claim 1, wherein MALP-2 is in the form of an aqueous solution, suspension or emulsion that is suitable for inhalation.

## Revendications

1. Utilisation du S-[2,3-bispalmitoyloxy-(2RS)-propyl] cystéinyl-GNNDESNISFKEK(MALP-2) en vue de la préparation d'un agent destiné au traitement de métastases pulmonaires de tumeurs extrapulmonaires.

2. Utilisation selon la revendication 1, le MALP-2 étant présent sous la forme d'une solution, d'une suspension ou d'une émulsion aqueuse appropriée à l'inhalation.
